# EUROPÄISCHE PATENTANMELDUNG

(11) **EP 1 132 465 A1**
(43) Veröffentlichungstag der Anmeldung: **12.09.2001**
(21) Anmeldenummer: 01105760.1
(22) Anmeldetag: 08.03.2001
(51) Int. Cl.: C12N 5/00

(54) **Wirkstoffkombination zur Kultivierung von Zellen**

(30) Priorität: 11.03.2000 DE 10011998
(71) Anmelder: FORSCHUNGSZENTRUM JÜLICH GMBH, 52425 Jülich (DE); Becker, Sven, Dr., 63477 Maintal (DE); Tonn, Torsten, Dr., 60528 Frankfurt am Main (DE)
(72) Erfinder: Schmidt, Sebastian, 52428 Jülich (DE); Gätgens, Jochen, 52428 Jülich (DE); Biselli, Manfred, 52428 Jülich (DE); Wandrey, Christian, 52428 Jülich (DE); Tonn, Torsten, 60328 Frankfurt am Main (DE); Becker, Sven, 63477 Maintal (DE); Seifried, Erhard, 61462 Königstein (DE)

(57) **Zusammenfassung**

Die Erfindung betrifft eine Wirkstoffkombination sowie ein Verfahren zur Kultivierung von Zellen, die erfindungsgemäß zu einer Förderung des Wachstums der Zellen führt.

Erfindungsgemäß enthält die Wirkstoffkombination mindestens eine Komponente aus der Gruppe bestehend aus L-Serin, L-Asparagin, L-Glutamin und D-Glukose. Durch die Wirkstoffkombination und das Verfahren kommt es zu einer Erhöhung des Wachstums von Zellen und folglich zu einer Erhöhung der Zelldichte, wodurch eine erhöhte Produktivität und eine Verbesserung der Kultivierungstechnik der Zellen erreicht wird.

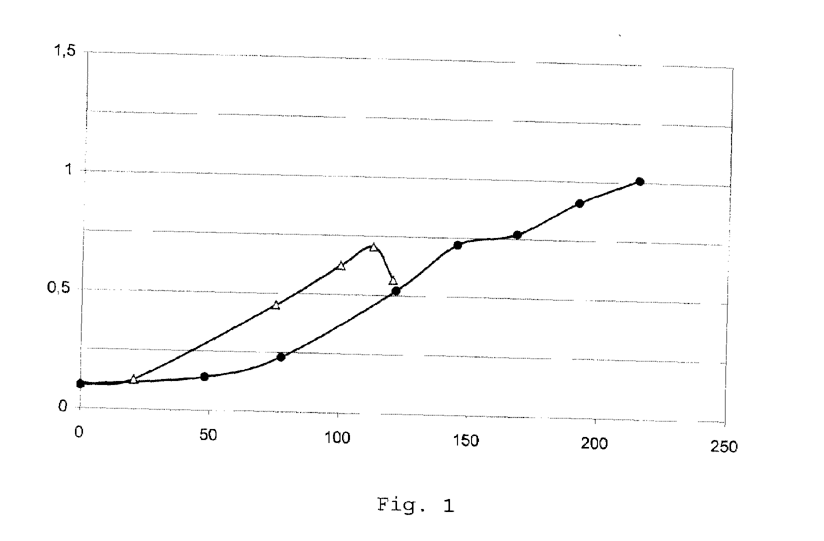

## Beschreibung

Die Erfindung betrifft eine Wirkstoffkombination zur Kultivierung von Zellen nach dem Oberbegriff des Anspruchs 1, ein Verfahren zur Kultivierung von Zellen nach dem Oberbegriff des Anspruchs 8 sowie ein Verfahren zur Herstellung einer Wirkstoffkombination nach dem Oberbegriff des Anspruchs 12.

Die Kultivierung von Zellen dient dazu, die Zellen selbst oder ihre Stoffwechselprodukte zu nutzen. Das optimale Wachstum der Zellen während der Kultivierung hängt von vielen verschiedenen Faktoren ab, wie z. B. der Sauerstoffkonzentration, dem pH-Wert, der Temperatur und verfügbaren Nährstoffen. Nährstoffe, die für das Zellwachstum benötigt werden, werden in Form des Mediums den Zellen zur Verfügung gestellt. Elemente, die am Zellaufbau der Zellen beteiligt sind, müssen in einer verwertbaren Verbindung im Medium enthalten sein. Die Zusammensetzung des Kulturmediums hängt daher von den Bedürfnissen des zu vermehrenden Organismus ab. Man unterscheidet zwischen synthetischen Medien, deren Inhaltsstoffe auf der Basis von Reinsubstanzen genau bekannt sind und Komplexmedien, deren Zusammensetzung schwanken kann und zum Teil nicht bekannt ist. Das Kulturmedium enthält neben Wasser mindestens eine für den Organismus assimilierbare Kohlenstoff- und Stickstoffquelle. Weiterhin wichtig ist auch das Vorhandensein einer Phosphat- und Schwefelquelle, Mineralstoffe sowie eventuell benötigte Wuchsstoffe und Vitamine.

Mit dem Begriff Kultivierung soll die Vermehrung und Haltung von Mikroorganismen, Organen, Geweben oder Einzelzellen, die von Pflanzen, Tieren oder Menschen abstammen, *in vitro* sowie auch *in vivo* umfaßt werden. Bei der Kultivierung von Zellen mit handelsüblichen Medien (z.B. Medium X-VIVO 10, BioWhittaker Europe, Taufkirchen) wird nur eine relativ geringe Zelldichte in der Kultur erreicht, d. h. daß die Kulturbedingungen zu einer Wachstumslimitierung führen. Die geringe Zelldichte wirkt sich nachteilig auf den Kultivierungsprozeß aus. Zum einen kann es zu Kontaminationen der Zellkultur mit unerwünschten Fremdorganismen kommen. Zum anderen ist bei geringen Zelldichten die Raum-Zeit-Ausbeute des Kultivierungsverfahrens gering.

Es ist daher die Aufgabe der Erfindung, einen Wirkstoff und ein Verfahren zu schaffen, mit dem eine Wachstumslimitierung aufgehoben und eine höhere Zelldichte erreicht werden kann als dies mit konventionellen Kulturmethoden erreicht wird, sowie ein Herstellungsverfahren für die Wirkstoffkombination zu entwickeln.

Ausgehend vom Oberbegriff des Anspruchs 1 wird die Aufgabe erfindungsgemäß gelöst mit den im kennzeichnenden Teil des Anspruchs 1 angegebenen Merkmalen sowie ausgehend vom Oberbegriff des Anspruchs 8 mit den im kennzeichnenden Teil des Anspruchs 8 und weiterhin ausgehend vom Oberbegriff des Anspruchs 12 mit den im kennzeichnenden Teil des Anspruchs 12 angegebenen Merkmalen.

Mit der erfindungsgemäßen Wirkstoffkombination, dem Verfahren zur Kultivierung der Zellen sowie dem Herstellungsverfahren der Wirkstoffkombination ist es nunmehr möglich, bei der Kultivierung von Zellen eine höhere Zelldichte zu erreichen, als dies mit konventionellen Kulturmethoden möglich ist.

Vorteilhafte Weiterbildungen sind in den Unteransprüchen angegeben.
Im folgenden soll die Erfindung beispielhaft erläutert werden.

Figur 1 zeigt beispielhaft das Wachstumsverhalten einer Zellkultur unter Verwendung der erfindungsgemäßen Wirkstoffkombination.

Es zeigt Figur 1: Einen Kulturverlauf einer Zellkultur mit und ohne Wirkstoffkombination.
In Figur 1 bildet die Abszisse X die Zeitachse in Stunden(h) und die Ordinate Y gibt die Zellkonzentration in Zellzahl ^{*} 10⁶ pro ml wieder. Der Versuchsansatz mit Zusatz der Wirkstoffkombination wird durch die geschlossenen Kreise (•)symbolisiert. Der Kontrollansatz ohne Zusatz der Wirkstoffkombination wird durch Dreiecke symbolisiert(Δ).

Tabelle 1 zeigt die sich insgesamt ergebenden Konzentrationen des Mediums mit zugesetzter Wirkstoffkombination und erweiterte mögliche Konzentrationsbereiche.

Bei der Kultivierung der Zellen wird dem handelsüblichen Basiskulturmedium die erfindungsgemäße Wirkstoffkombination zugesetzt.
Das Basiskulturmedium kann ein Minimalmedium sein, welches mindestens eine Kohlenstoffquelle, Spurenelemente und Salze, wie z.B. NH4C1, K2HPO4, MgSO4, FeSO4 und CaCl2 enthalten kann. Kulturmedien für tierische oder humane Zellen enthalten im allgemeinen Glucose, Galactose, Aminosäuren, B-Vitamine, Cholin, Inosit und anorganische Salze. Ein solches Medium wird als Minimalmedium bezeichnet und muß zur Förderung der Zell-Proliferation mit Blutserum ergänzt werden. Bei serumfreien Medien werden die wachstumsstimulierenden Substanzen der Seren z. B. durch Insulin, Wachstumshormone, epidermale Wachstumsfaktoren, Interleukine, Prostagladine, Hydrocortison u.a. ersetzt.
Das Basiskulturmedium kann aber auch ein Komplexmedium sein, das z. B. Serum, Albumine, Hefeextrakt, Hefeautolysat, Pepton oder Fleischextrakt enthalten kann oder auch Molke, Melasse, Maisquellwasser oder Sojabohnenextrakt beinhalten kann.
Die erfindungsgemäße Wirkstoffkombination kann sowohl in fester als auch in gelöster Form eingesetzt werden. Der Einsatz der Wirkstoffkombination eignet sich zur Verwendung in einer viskosen Trägermatrix (z. B. Agar, Methylcellulose, Silikagel) genauso wie in flüssigen Medien (z. B. wässrig gelöste Medien). Beispielsweise kann die Wirkstoffkombination in Form einer hochkonzentrierten Stammlösung vorbereitet werden und bei Einsatz sterilfiltriert dem jeweiligen handelsüblichen Basismedium zugesetzt werden. Eine weitere Möglichkeit besteht darin, die Komponenten in Pulverform einzuwiegen und dem Basismedium zuzusetzen. Zur Herstellung des Kulturmediums kann die Wirkstoffkomponente daher in wässrig gelöster Form dem ebenfalls wässrig gelösten Kulturmedium zugefügt werden bzw. in Pulverform zusammen mit den übrigen festen Komponenten des Kulturmediums eingesetzt werden. Weiterhin ist auch der Einsatz in viskosen Kulturmedien möglich. Die Zugabe der Wirkstoffkombination zu unterschiedlichen Zeitpunkten der Kultivierung oder Fermentation der Zellen führt ebenfalls zur gewünschten wachstumsfördernden Wirkung. Auch der Einsatz der Wirkstoffkombination ohne Verwendung anderer Medienkomponenten ist möglich.

Die einzelnen Komponenten Serin, Asparagin und Glutamin der Wirkstoffkombination können einzeln oder in jeder beliebigen Kombination eingesetzt werden. In einer bevorzugten Ausführungsform werden alle drei Aminosäuren zusammen eingesetzt. Durch Zugabe von Glucose wird noch eine weitere Erhöhung der Zelldichte ermöglicht.

Die einzelnen Komponenten L-Serin, L-Asparagin, L-Glutamin und D-Glucose der Wirkstoffkombination haben sich als wachstumslimitierende Faktoren erwiesen. Mit Hilfe ihrer Zugabe kommt es zur Erhöhung der Zelldichte und zur Vermeidung der Wachstumslimitierung. Die Komponente L-Serin führt bereits ab einer Konzentration von 0,45 mM zu einem verbesserten Zellwachstum. Die positive Wirkung von L-Asparagin setzt bereits bei 0,25mM ein und die von L-Glutamin ab einer Konzentration von 4,50 mM. D-Glucose bewirkt ab einer Konzentration von 27,0 mM das Aufheben der Wachstumslimitierung. Die einzelnen Komponenten können in unterschiedlichen Konzentrationen bis zur jeweiligen Löslichkeitsgrenze eingesetzt werden (s. Tab. 1, möglicher Bereich). Eine Erhöhung der Zellkonzentration wurde bereits durch Zugabe der Wirkstoffkomponenten in folgenden Konzentrationen erreicht:

| | |
|---|---|
| L-Serin | 0,45 bis 5,0 mM |
| L-Asparagin | 0,25 bis 5,0 mM |
| L-Glutamin | 4,50 bis 10,0 mM |
| | |
| D-Glucose | 27,0 bis 50 mM |

Eine weitere Erhöhung der Zellkonzentration gegenüber handelsüblichen Medien wird durch die Zugabe der Komponenten in folgenden Konzentrationen erreicht:

| | |
|---|---|
| L-Serin | 0,45 bis 10,0 mM |
| L-Asparagin | 0,25 bis 10,0 mM |
| L-Glutamin | 4,50 bis 20,0 mM |
| | |
| D-Glucose | 27 mM bis 100 mM |

In einer besonders bevorzugten Ausführung werden die Komponenten in folgenden Konzentrationen eingesetzt:

| | |
|---|---|
| L-Serin | 1,90 mM |
| L-Asparagin | 1,19 mM |
| L-Glutamin | 5,50 mM |
| | |
| D-Glucose | 36,1 mM |

Die Verwendung der Wirkstoffkombination eignet sich zur Induktion des Wachstums von humanen Zellen. Bevorzugt sind hier vor allem humane Primärzellen wie auch die sogenannten natürlichen Killerzellen. Möglich ist jedoch auch eine wachstumsfördernde Wirkung auf Säugerzellen oder auch Zellen von Mikroorganismen.
Durch den Einsatz der Wirkstoffkombination wird die Wachstumslimitierung aufgehoben und damit eine höhere Zelldichte erzielt. Dies wirkt sich zum einen positiv auf die Raum-Zeit-Ausbeute des Verfahrens aus, und zum anderen führt es zur Verringerung der Kontaminationsgefahr mit unerwünschten Fremdorganismen.

### Ausführungsbeispiel:

Die humane natürliche Killerzelle NK-92 wurde in X-VIVO 10 Medium und in X-VIVO 10 mit Zugabe der Wirkstoffkombination, wobei eine Endkonzentration von 1,90 mM für L-Serin, 1,19 mM für L-Asparagin, 5,50 mM für L-Glutamin und 36,10 mM für D-Glucose (s. auch Tab. 1)eingestellt wurde, in 100 ml Volumen in einem 500 ml Spinnergefäß (Techne, Wertheim/Main) als ungefütterter Kultur im Brutschrank (37°C, 100% Luftfeuchtigkeit) kultiviert. In beiden Fällen wurden als Wachstumsfaktoren das Zytokin Interleukin-2 (IL-2, Proleukin, Chiron GmbH, Ratingen) zugegeben.

Während in reinem X-VIVO 10 + IL-2 eine maximale Zelldichte von 7,05^{*}10⁵ (Zellen/ml) erreicht wurde, konnte durch die neue Medienrezeptur die Dichte um 43% auf 1,01^{*}10⁶ (Zellen/ml) gesteigert werden (s. Fig. 1).

**Tab. 1**

| **Medien-kompo- nente:** | **X-Vivo 10 lt. Analyse 07.04.99 [= IMDM]** (mM) | **Medium + Wirkstoff- kombination** (mM) | **bevorzugter Bereich** (mM) | **möglicher Bereich** (mM) |
|---|---|---|---|---|
| **L-Serin** [Mr = 105,09] | 0,40 | 1,90 | 0,45-5 | 0,45-2570 |
| **L-Asparagin * H**_{**2**}**O** [Mr = 150,14] | 0,19 | 1,19 | 0,25-5 | 0,25-233 |
| **L-Glutamin** [Mr = 146,10] | 4,00 | 5,50 | 4,5-10 | 4,5-178 |
| **D-Glucose** [Mr = 180,20] | 25 | 36,10 | 27-50 | 27-1000 |

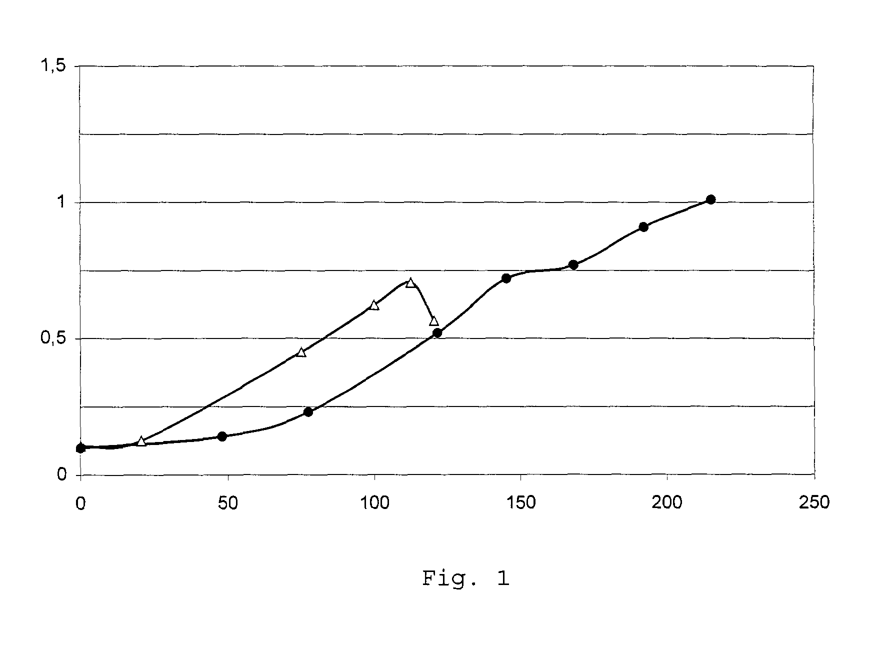

## Patentansprüche

1. Zellwachstum fördernde Wirkstoffkombination,
**dadurch gekennzeichnet,**
**daß** die Wirkstoffkombination mindestens eine Komponente aus der Gruppe bestehend aus L-Serin, L-Asparagin und L-Glutamin enthält.

2. Wirkstoffkombination nach Anspruch 1,
**dadurch gekennzeichnet,**
**daß** die Wirkstoffkombination alle drei Aminosäuren L-Serin, L-Asparagin und L-Glutamin enthält.

3. Wirkstoffkombination nach einem Ansprüche 1 oder 2,
**dadurch gekennzeichnet,**
**daß** der Konzentrationsbereich für L-Serin zwischen > 0,4 und 2570 mM liegt, der von L-Asparagin zwischen > 0,19 und 233 mM und der von L-Glutamin zwischen > 4,0 und 178 mM liegt.

4. Wirkstoffkombination nach einem der Ansprüche 1 bis 3,
**dadurch gekennzeichnet,**
**daß** die Wirkstoffkombination D-Glucose umfaßt.

5. Wirkstoffkombination nach Anspruch 4,
**dadurch gekennzeichnet,**
**daß** der Konzentrationsbereich für D-Glucose zwischen > 25 und 1000 mM liegt.

6. Wirkstoffkombination nach einem der Ansprüche 1 bis 5,
**dadurch gekennzeichnet,**
**daß** die Wirkstoffkombination Wachstumsfaktoren umfaßt.

7. Wirkstoffkombination nach Anspruch 6,
**dadurch gekennzeichnet,**
**daß** die Wirkstoffkombination Zytokine umfaßt.

8. Verfahren zur Kultivierung von Zellen unter Verwendung einer Wirkstoffkombination
**dadurch gekennzeichnet,**
**daß** bei der Kultivierung die Wirkstoffkombination gemäß Anspruch 1 bis 7 eingesetzt wird.

9. Verfahren nach Anspruch 8,
**dadurch gekennzeichnet,**
**daß** bei der Kultivierung die Wirkstoffkombination in wässrig gelöster Form, in Verbindung mit einer viskosen Trägermatrix oder in fester Form eingesetzt wird.

10. Verfahren nach Anspruch 8 oder 9,
**dadurch gekennzeichnet,**
**daß** mit dieser Wirkstoffkombination das Wachstum humaner Zellen und Säugerzellen gefördert wird.

11. Verfahren zur Herstellung einer wachstumsfördernden Wirkstoffkombination
**dadurch gekennzeichnet,**
**daß** für diese Wirkstoffkombination mindestens eine Komponente aus der Gruppe bestehend aus L-Serin, L-Asparagin und L-Glutamin verwendet wird.

12. Verfahren nach Anspruch 11,
**dadurch gekennzeichnet,**
**daß** für diese Wirkstoffkombination alle drei Aminosäuren L-Serin, L-Asparagin und L-Glutamin verwendet werden.

13. Verfahren nach einem der Ansprüche 11 bis 12,
**dadurch gekennzeichnet,**
**daß** L-Serin in einem Konzentrationsbereich zwischen > 0,4 und 2570 mM eingesetzt wird, L-Asparagin in einem Konzentrationsbereich zwischen > 0,19 und 233 mM und L-Glutamin in einem Konzentrationsbereich zwischen > 4,0 und 178 mM eingesetzt wird.

14. Verfahren nach einem der Ansprüche 11 bis 13,
**dadurch gekennzeichnet,**
**daß** für diese Wirkstoffkombination D-Glucose verwendet wird.

15. Verfahren nach einem der Ansprüche 11 bis 14,
**dadurch gekennzeichnet,**
**daß** D-Glucose in einem Konzentrationsbereich zwischen > 25 und 1000 mM eingesetzt wird.

16. Verfahren nach einem der Ansprüche 11 bis 15,
**dadurch gekennzeichnet,**
**daß** die Wirkstoffkombination in wässrig gelöster Form in Verbindung mit einer viskosen Trägermatrix sowie in fester Form vorliegen kann.
